# EUROPEAN PATENT APPLICATION

(11) **EP 1 475 103 A1**
(43) Date of publication of application: **10.11.2004**
(21) Application number: 03731835.9
(22) Date of filing: 24.01.2003
(51) Int. Cl.: A61K 45/00, A61K 31/09, A61P 13/12

(54) **REMEDIES FOR CHRONIC KIDNEY DISEASES**

(30) Priority: 25.01.2002 JP 2002017474
(71) Applicant: TAISHO PHARMACEUTICAL CO., LTD, Tokyo 170-8633 (JP)
(72) Inventor: MIYATA, Noriyuki c/o TAISHO PHARMACEUT. CO., LTD., Toshima-ku, Tokyo 170-8633 (JP); OKUYAMA, Shigeru c/o TAISHO PHARMACEUT. CO., LTD., Toshima-ku, Tokyo 170-8633 (JP); TAKAHASHI, Teisuke c/o TAISHO PHARMAC. CO., LTD., Toshima-ku, Tokyo 170-8633 (JP)
(74) Representative: HOFFMANN EITLE
(86) International application number: PCT/JP2003/000675
(87) International publication number: WO 2003/061700

(57) **Abstract**

A therapeutic agent for chronic kidney diseases, which comprises as an active ingredient a compound capable of inhibiting Na⁺/Ca²⁺ exchanger 1.

## Description

### TECHNICAL FIELD

The present invention relates to novel therapeutic agents for chronic kidney diseases.

### BACKGROUND ART

Intracellular free Ca²⁺ is an important ion for controlling the contraction of cardiac muscle and various smooth muscles, the release of neurotransmitters, and the expression of genes. The concentration of such Ca²⁺ ion is regulated by Ca²⁺ pumps, Ca²⁺ channels and/or Na⁺/Ca²⁺ exchangers (NCX) present in the plasma membrane and the sarcoplasmic reticulum membrane. Among them, Na⁺/Ca²⁺ exchangers play a particularly important role in the contraction and relaxation of cardiac muscle and vascular smooth muscle (Ann. Rev. Physiol., vol. 52, p. 467 (1990)). At present, three NCX genes have been isolated and identified from mammals. Moreover, it is known that NCX1 protein is expressed at high levels in the brain, heart and kidney, NCX2 protein is expressed primarily in the brain and also expressed, but slightly, in visceral smooth muscle, and NCX3 protein is expressed primarily in the brain and also expressed, but slightly, in skeletal muscle (Jpn. J. Circ. Res, vol. 24, no. 3, p. 101 (2001); Am. J. Physiol., 272, C1250-C1261 (1997)).

As for NCX inhibitors, isothiourea derivatives such as 2-[2-[4-[nitrobenzyloxy] phenyl] ethyl] isothio - ureamethanesulfonate (K-BR7943) and phenoxyaniline derivatives such as 2-[4-[(2,5-difluorophenyl)methoxy] phenoxy]-5-ethoxyaniline (SEA0400) have been reported, and K-BR7943 has been confirmed to have efficacy in acute mycardial infarction models as well as brain and kidney ischemia/reperfusion models (J. Pharmacol. Exp. Ther., vol. 296, p. 412 (2001)). However, there is no report about the application of NCX inhibitors as therapeutic agents for chronic kidney diseases.

### DISCLOSURE OF THE INVENTION

The inventors of the present invention investigated the NCX-inhibiting activity of K-BR7943, SEA0400 and other compounds by using Na⁺/Ca²⁺ exchangers (NCX) prepared from brain, heart and kidney. As a result, it has been found that phenoxyaniline derivatives (including SEA0400) and phenoxypyridine derivatives selectively inhibit NCX derived from the heart and kidney, as compared with NCX derived from brain tissue.

In addition, previous studies have reported that the above-mentioned compounds had little effect on other receptors, channels and transporters, at a concentration sufficient to inhibit NCX (J. Pharmacol. Exp. Ther., vol. 298, p. 249 (2001)).

In view of the foregoing, phenoxyaniline derivatives and the like are found to have high selectivity for NCX1.

With the aim of elucidating the relationship between NCX1 and diseases or their treatment, the inventors of the present invention made further investigation in various disease models (e.g., chronic kidney failure/glomerulosclerosis model, diabetic rat) using the above NCX1-selective inhibitors. As a result, it has been found that inhibition of NCX1 is effective for the renal protection in chronic kidney failure, glomerulosclerosis and diabetic nephropathy. These findings led to the completion of the present invention.

Namely, the present invention is directed to a therapeutic agent for chronic kidney diseases, which comprises as an active ingredient a compound capable of inhibiting Na⁺/Ca²⁺ exchanger 1.

Further, the present invention is directed to a therapeutic agent for chronic kidney diseases, which comprises as an active ingredient a 2-phenoxyaniline derivative of Formula (1): 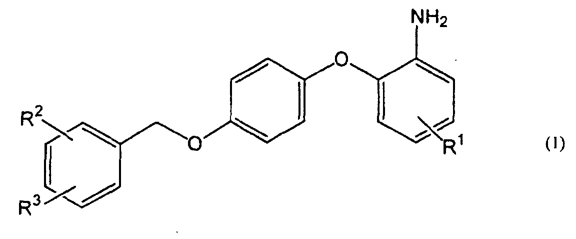 [wherein R¹ represents a hydrogen atom or a C₁-C₆ alkoxy group, R² represents a halogen atom or a nitro group, and R³ represents a hydrogen atom or a halogen atom] or a pharmaceutically acceptable salt thereof.

### BEST MODE FOR CARRYING OUT THE INVENTION

In the present invention, NCX1 inhibiting compounds are not limited as long as they inhibit kidney-derived NCX1, preferred are those which produce greater than 50% inhibition at a concentration of 3 µM when assayed by the test described later (Reference Example 3). In terms of avoiding side effects, more preferred are compounds capable of specifically inhibiting NCX1.

The compound capable of specifically inhibiting NCX1 means a compound that does not substantially inhibit other receptors, channels and transporters at a concentration sufficient to inhibit NCX1. More specifically, for example, when used at a concentration of 3 µM, such a compound preferably does not cause greater than 50% inhibition of Ca²⁺ channels; Na⁺ channels, K⁺ channels, Na⁺/H⁺ transporters, norepinephrine transporters, Na⁺,K⁺-ATPase, Ca²⁺-ATPase, phospholipase A₂, phospholipase C, 5-lipoxygenase, inducible nitric-oxide synthetase, constitutive nitric-oxide synthetase, Adenosine receptors, Adrenergic receptors, Glutamate receptors, Bradykinin receptors, LTB4 receptors or PAF receptors. It should be noted that procedures for measurement using these individual ion channels, enzymes and receptors are disclosed in J. Pharmacol. Exp. Ther., vol. 298, p. 249 (2001) and references cited therein.

Examples of the compound capable of specifically inhibiting NCX1 include phenoxyaniline derivatives and phenoxypyridine derivatives.

Preferred are compounds of Formula (2): 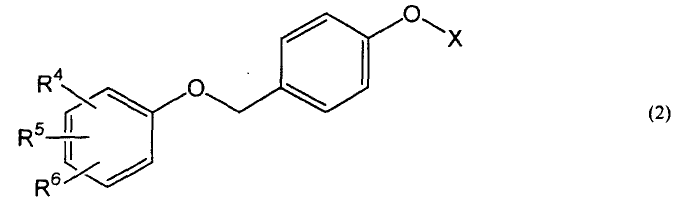 [wherein R⁴, R⁵ and R⁶, which may be identical or different, each represent a hydrogen atom or a halogen atom, X represents: 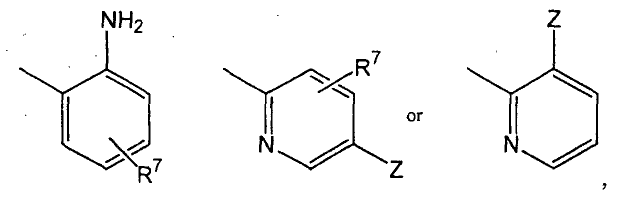 R⁷ represents a hydrogen atom, a substituted or unsubstituted C₁-C₆ alkyl group or a substituted or unsubstituted C₁-C₆ alkoxy group, Z represents a nitro group, an amino group or a NHC(O)CH₂R⁸ group, R⁸ represents a hydrogen atom, a substituted or unsubstituted C₁-C₆ alkyl group, a substituted or unsubstituted C₁-C₆ alkoxy group, a halogen atom, a hydroxy group, a C₂-C₇ acyloxy group, NR⁹R¹⁰ or 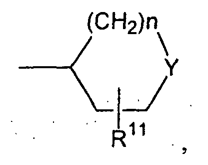 R⁹ and R¹⁰, which may be identical or different, each represent a hydrogen atom, a substituted or unsubstituted C₁-C₆ alkyl group or an N-methyl-4-piperidinyl group, R¹¹ represents a hydrogen atom, a hydroxy group or a C₂-C₇ alkoxycarbonyl group, Y represents a methylene group, an epoxy group, a thio group or a NR¹² group, n represents an integer of 1 to 4, and R¹² represents a hydrogen atom, a substituted or unsubstituted C₁-C₆ alkyl group or a substituted or unsubstituted phenyl group] or a pharmaceutically acceptable salt thereof.

In terms of NCX1-inhibiting activity, more preferred are 2-phenoxyaniline derivatives of Formula (1): 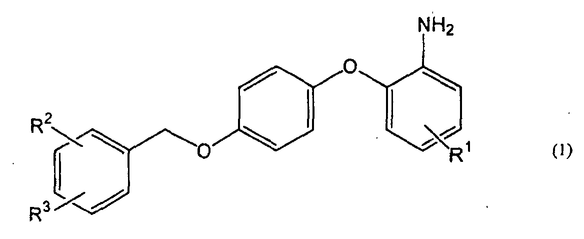 [wherein R¹ represents a hydrogen atom or a C₁-C₆ alkoxy group, R² represents a halogen atom or a nitro group, and R³ represents a hydrogen atom or a halogen atom] or a pharmaceutically acceptable salt thereof.

In Formulae (1) and (2), a C₁-C₆ alkoxy group means a linear or branched alkoxy group containing 1 to 6 carbon atoms. Examples include a methoxy group, an ethoxy group, a propoxy group, an isopropoxy group, a butoxy group, an isobutoxy group, a sec-butoxy group, a tert-butoxy group, a pentyloxy group, an isopentyloxy group, a neopentyloxy group, a tert-pentyloxy group, a 1-methylbutoxy group, a 2-methylbutoxy group, a 1,2-dimethylpropoxy group, a hexyloxy group and an isohexyloxy group.

Examples of a substituent for the substituted C₁-C₆ alkoxy group include a chloro group, a fluoro group, a nitro group, an amino group, a dimethylamino group, a carboxyl group, a methoxycarbonyl group, an ethoxycarbonyl group, a phenyl group, a hydroxy group, a cyano group and a carbamoyl group.

A halogen atom refers to a fluorine atom, a chlorine atom, a bromine atom or an iodine atom.

A C₁-C₆ alkyl group means a linear or branched alkyl group containing 1 to 6 carbon atoms. Examples include a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a 1-methylbutyl group, a 2-methylbutyl group, a 1,2-dimethylpropyl group, a hexyl group and an isohexyl group.

Examples of a substituent for the substituted C₁-C₆ alkyl group include a chloro group, a fluoro group, a nitro group, an amino group, a dimethylamino group, a carboxyl group, a methoxycarbonyl group, an ethoxycarbonyl group, a phenyl group, a methoxy group, an ethoxy group, a hydroxy group, a cyano group and a carbamoyl group.

A C₂-C₇ acyloxy group means a linear or branched acyloxy group containing 2 to 7 carbon atoms, whose acyl moiety may be cyclic or may contain an aromatic group. Examples include an acetoxy group, a propionyloxy group, an isopropionyloxy group, a cyclohexyloxy group and a benzoyloxy group.

A C₂-C₇ alkoxycarbonyl group means a linear or branched alkoxycarbonyl group containing 2 to 7 carbon atoms, whose alkoxyl moiety may be cyclic or may contain an aromatic group. Examples include a methoxycarbonyl group, an ethoxycarbonyl group, a propoxycarbonyl group, an isopropoxycarbonyl group, a butoxycarbonyl group, an isobutoxycarbonyl group, a sec-butoxycarbonyl group, a tert-butoxycarbonyl group, a pentyloxycarbonyl group, an isopentyloxycarbonyl group, a neopentyloxycarbonyl group, a tert-pentyloxycarbonyl group, a 1-methylbutoxycarbony] group, a 2-methylbutoxycarbonyl group, a 1,2-dimethylpropoxycarbonyl group, a hexyloxycarbonyl group and an isohexyloxycarbonyl group.

Examples of a substituent for the substituted phenyl group include a chloro group, a fluoro group, a nitro group, an amino group, a dimethylamino group, a carboxyl group, a methoxycarbonyl group, an ethoxycarbonyl group, a methyl group, an ethyl group, a methoxy group, an ethoxy group, a hydroxy group, a cyano group and a carbamoyl group.

Examples of compounds having an excellent activity for kidney protect are the compound shown below (SEA0400) 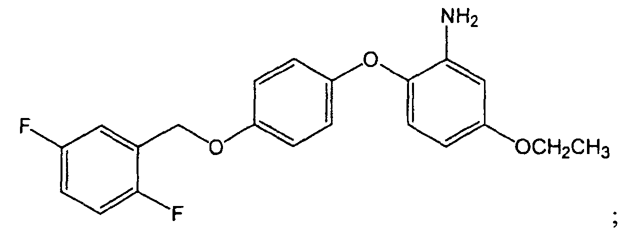 and
the compound shown below (SEA0064).

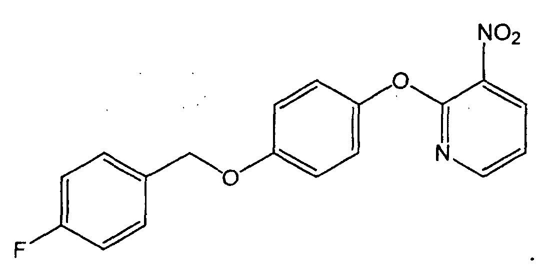

Moreover, to avoid side effects, such compounds preferably produce stronger inhibition on NCX1 than on NCX2 and NCX3. For example, the compounds that have smaller values of
IC₅₀ (renal cortex-derived) / IC₅₀ (brain-derived), and
IC₅₀ (renal cortex-derived) / IC₅₀ (cardiac sarcolemma-derived) , when measured as described later, than SEA 0400 are preferred.

It should be noted that the compounds of Formulae (1) and (2) can be synthesized according to the procedures as described in WO98/43943, WO99/20598, Japanese Laid-Open Patent *Hei* 10-265460, Japanese Laid-Open Patent *Hei* 10-218844, Japanese Laid-Open Patent *Hei* 11-49752 and Japanese Laid-Open Patent *Hei* 11-92454.

As used herein, the therapeutic agent for a chronic kidney disease of the present invention means a therapeutic agent for a chronic kidney disease which deteriorates the physiological function of kidney including chronic kidney failure, glomerulosclerosis, diabetic nephropathy, nephrosclerosis, chronic nephritis, chronic glomerulonephritis, polycystic kidney disease and the like, and can achieve excellent kidney protective activity such as decreasing the protein and albumin in urine.

The therapeutic agent of the present invention can be prepared as a pharmaceutical composition in any desired dosage form for oral or parenteral use (e.g., tablets, pills, capsules, granules, dry syrups, injectable preparations), in combination with known carriers, diluents and so on, as appropriate.

Solid preparations can be prepared by stirring granulation, fluidized-bed granulation or milling granulation using various additives such as excipients, disintegrating agents, binders, lubricants and coating bases.

If necessary, it is also possible to add other additives such as antioxidants, coating agents, coloring agents, flavoring agents, surfactants and plasticizers.

The dose of the active ingredient in the pharmaceutical preparation of the present invention will vary depending on age, body weight, dosage form, and so on, but the usual dose to an adult is 0.1 to 1000 mg/day, which can be administrated once or several times a day.

The present invention will now be described with reference to the following Formulation Examples and Test Examples, which are not intended to limit the scope of the invention.

| Formulation Example 1 | |
|---|---|
| SEA0400 | 50 mg |
| Lactose | 40 mg |
| Corn starch | 49.75 mg |
| Crystalline cellulose | 17 mg |
| Carmellose calcium | 17 mg |
| Hydroxypropylcellulose | 5.25 mg |
| Magnesium stearate | 1 mg |
| Total | 180 mg |

SEA0400, lactose, corn starch, crystalline cellulose and carmellose calcium were mixed uniformly, followed by addition of a 10% aqueous hydroxypropylcellulose solution. After kneading and drying, the resulting granules were passed through a 30M sieve to give uniform granules, which were then supplemented with magnesium stearate and tabletted into tablets.

| Formulation Example 2 | |
|---|---|
| SEA0064 | 50 mg |
| Lactose | 40 mg |
| Corn starch | 49.75 mg |
| Crystalline cellulose | 17 mg |
| Carmellose calcium | 17 mg |
| Hydroxypropylcellulose | 5.25 mg |
| Magnesium stearate | 1 mg |
| Total | 180 mg |

SEA0064, lactose, corn starch, crystalline cellulose and carmellose calcium were mixed uniformly, followed by addition of a 10% aqueous hydroxypropylcellulose solution. After kneading and drying, the resulting granules were passed through a 30M sieve to give uniform granules, which were then supplemented with magnesium stearate and tabletted into tablets.

### Reference Example 1 Method of measuring for brain microsomal Na⁺/Ca²⁺ exchanger

Brain microsomes (1.5 mg/ml) obtained from 8-week-old rats were pre-treated with 160 mM NaCl-containing buffer to cause Na loading into membrane vesicles. This suspension was diluted 50-fold with 20 µM ⁴⁵CaCl₂-containing buffer to induce ⁴⁵Ca uptake, followed by dilution with the buffer (0°C) to stop the reaction. The membrane vesicles were immediately collected on a nitrocellulose filter. Subsequently, ⁴⁵Ca trapped inside the membrane vesicles was determined by liquid scintillation counting. The above assay for Na⁺/Ca²⁺ exchange activity in brain microsomes was performed according to the procedures described in J. Biol. Chem., vol. 257, p. 5111 (1982).

### Reference Example 2 Method of measuring for canine cardiac sarcolemmal vesicle Na⁺/Ca²⁺ exchanger

Canine cardiac sarcolemmal vesicles (0.5 mg/ml) were prepared by centrifugal fractionation as described in Methods enzymology, vol. 157, p. 85 (1988) and suspended in Solution A (20 mM MOPS-Tris (pH 7.4), 160 mM NaCl or KCl), followed by incubation at room temperature for about one hour to cause Na or K loading into the vesicles. This suspension was diluted 50-fold with 20 µM ⁴⁵CaCl₂-containing buffer to induce ⁴⁵Ca uptake, followed by dilution with the buffer (0°C) to stop the reaction. The membrane vesicles were immediately collected on a nitrocellulose filter. Subsequently, ⁴⁵Ca trapped inside the membrane vesicles was determined by liquid scintillation counting. The Na⁺/Ca²⁺ exchange activity was evaluated by subtracting the value obtained for K loading from the value obtained for Na loading. The above assay for Na⁺/Ca²⁺ exchange activity in canine cardiac sarcolemmal vesicles was performed according to the procedures described in J. Biol. Chem., vol. 257, p. 5111 (1982).

### Reference Example 3 Preparation of rat renal cortex-derived BLMVs (basolateral membrane vesicles) and method of measuring for their Na⁺/Ca²⁺ exchange activity

### (Preparation of BLMVs)

BLMVs were prepared from rat renal cortex and assayed for Na⁺/Ca²⁺ exchange activity according to the procedures described in Am. J. Physiol., vol. 266, p. F785 (1994).

After being excised from rats, the kidneys were placed in ice-cold sucrose buffer (0.25 mM sucrose, 0.1 mM PMSF, 10 mM Tris-HCl (pH 7.6)) and decapsulated. The isolated cortex was then finely minced in the sucrose buffer and homogenized sequentially with a Dounce-type homogenizer and a Polytron-type homogenizer, followed by centrifugation at 2500 g for 15 minutes to collect the supernatant. Centrifugation was repeated at 24000 g for 20 minutes to collect the white fluffy portion of the pellet. After further addition of the sucrose buffer, the collected fraction was homogenized with a Dounce-type homogenizer, supplemented with Percoll and then centrifuged at 30000 g for 35 minutes to collect the middle layer. After addition of buffer (100 mM KCl, 100 mM mannitol, 5 mM HEPES-Tris (pH 7.4)), centrifugation was carried out at 34000 g for 30 minutes to collect the loose white pellet (BLMVs). The pellet was further suspended in the KCl-mannitol buffer and then centrifuged at 34000 g for 30 minutes to collect the precipitate, which was used for activity assay.

### (Method of measuring for Na⁺/Ca²⁺ exchanger (Na-dependent ⁴⁵Ca uptake))

The BLMVs thus prepared were equilibrated at 37°C for 10 minutes in pre-equilibration buffer (100 mM NaCl, 40 mM KCl, 1 mM MgSO₄, 10 mM glucose, 5 mM HEPES-Tris (pH 7.4)) and then centrifuged at 20000 g for 5 minutes to collect the precipitate, which was then resuspended in the pre-equilibration buffer. Centrifugation was repeated to collect the precipitate, followed by resuspension in the pre-equilibration buffer. The resulting BLMV suspension was diluted 20-fold with external medium (100 mM choline chloride, 40 mM KCl, 1 mM MgSO₄, 10 mM glucose, 5 mM HEPES-Tris (pH 7.4), 25 µM valinomycin, 10 µM CaCl₂, 1 mCi/l⁴⁵CaCl₂) to start uptake. After reaction at 25°C for a given period of time, 2 ml stop solution (ice-cold 150 mM KCl) was added to stop the reaction, and the reaction mixture was immediately filtered through an ultrafiltration membrane (0.45 µm nitrocellulose filter) to collect BLMVs on the filter. Subsequently, the filter was washed twice with 2 ml stop solution and then the amount of ⁴⁵Ca trapped inside the BLMVs was determined by using liquid scintillation method.

**Table 1**

| Inhibitory activity against Na⁺/Ca²⁺ exchange (Na-dependent ⁴⁵Ca uptake) | | | |
|---|---|---|---|
| (IC₅₀ value: µM) | | | |
| | Brain | Cardiac sarcolemma | Renal cortex |
| K-BR7943 | 11.0 | 7.0 | 6.8 |
| SEA0400 | 0.2 | 0.09 | 0.02 |

Likewise, SEA0064 was also confirmed to show an inhibitory activity of about 0.07-fold of SEA0400, by measuring for renal cortex-derived Na⁺/Ca²⁺ exchange activity.

### Test Example 1: Kidney protective activity of Na⁺/Ca²⁺ exchange system inhibitor in Dahl-salt sensitive rat

This test was conducted following the method described in J. Cardiovascular Pharmacology, Vol. 23, p. 970 (1994). Specifically, procedures are as follows.

### <Method>

Dahl-salt sensitive (Dahl-S) rats and Dahl-salt resistant (Dahl-R) rats as control animals (male, 7 weeks old) were used for the experiment. The rats were divided into 3 groups (6 individuals for each group) for the test.
Group I: Dahl-R + 4% NaCl chow + solvent orally administered (twice a day)
Group II: Dahl-S + 4% NaCl chow + solvent orally administered (twice a day)
Group III: Dahl-S + 4% NaCl chow + SEA0400 10 mg/kg orally administered (twice a day)

Each animal was fed with a 4% NaCl high-salt-containing chow (whereas normal chow contains 0.6% NaCl) and water ad libitum. The solvent and SEA0400 were orally administered twice a day (first time at 8:30 to 9:30 and second time at 16:30 to 17:30) for consecutive 6 weeks. In 6 weeks after the administration started, each of the rats was placed in a metabolic cage where urine was taken so that the amounts of protein and albumin in urine were measured to observe the effect on the kidney function. Determination of the amounts of protein and albumin in urine was performed following the method described in Bunseki Kagaku, Vol. 32, pp. E379-E386 (1983) and Hypertension, Vol. 40, No. 6, pp. 834-839 (2002) using an autoanalyzer (HITACHI Automatic Analyzer 7060).

The amounts of urinary protein excretion in 6 weeks after the test started were 23±2 mg/day for Group I and 144±11 mg/day for Group II but 91±12 mg/day for SEA0400 administered group (Group III), which value was significantly lower than that of the solvent administered group (Group II).

The amounts of urinary albumin excretion were 2±1 mg/day for Group I and 109±14 mg/day for Group II but 58±8 mg/day for SEA0400 administered group (Group III), which value was significantly lower than that of the solvent administered group (Group II).

These results demonstrated that SEA0400 significantly inhibited the increase in urinary protein and albumin excretion observed in Dahl-S rats and exhibited kidney protective activity.

Test Example 2: Kidney protective activity of Na⁺/Ca²⁺ exchanger inhibitor in spontaneous diabetic rats (ZSF-1)

### <Method>

ZSF-1 rats are spontaneous diabetic rats and are known to exhibit renal dysfunction (Renal Failure, Vol. 22, No. 4, pp. 387-406 (2000)). ZSF-1 rats and ZSF-1/lean rats which were control animals (8 weeks old) obtained from The Jackson Laboratory were used for this test. The rats were divided into 5 groups (8 individuals for each group) for the test.
Group I: ZSF-1/lean + solvent orally administered (twice a day)
Group II: ZSF-1 + solvent orally administered (twice a day)
Group III: ZSF-1 + SEA0400 1 mg/kg orally administered (twice a day)
Group IV: ZSF-1 + SEA0400 3 mg/kg orally administered (twice a day)
Group V: ZSF-1 + SEA0400 10 mg/kg orally administered (twice a day)

The solvent and SEA0400 were orally administered twice a day (first time at 8:30 to 10:30 and second time at 16:30 to 18:30) for consecutive 8 weeks. Eight weeks after the administration started, each of the rats was placed in a metabolic cage where urine was taken so that the amounts of protein and albumin in urine were measured to observe the effect on the kidney function. Determination of the amounts of protein and albumin was performed following the description of the above-mentioned references using an autoanalyzer (HITACHI 7060 Automatic Analyzer).

The amounts of urinary protein excretion in 8 weeks after the test started were 14±1 mg/day for Group I; 58±9 mg/day for Group II; 40±8 mg/day for Group III; 37±7 mg/day for Group IV; and 29 ± 5 mg/day for Group V, and the value of the group administered with 10 mg/kg of SEA0400 (Group V) was significantly lower than that of the solvent administered group (Group II).

The amounts of urinary albumin excretion were 1±0.1 mg/day for Group I; 28 ± 5 mg/day for Group II; 20±5 mg/day for Group III; 17 ± 5 mg/day for Group IV; and 9 ± 2 mg/day for Group V, and the value of the group administered with 10 mg/kg of SEA0400 (Group V) was significantly lower than that of the solvent administered group (Group II).

These results demonstrated that SEA0400 significantly inhibited the increase in urinary protein and albumin excretion as observed in spontaneous diabetic rats and exhibited kidney protective activity.

### Industrial Applicability

The present invention enables to provide therapeutic agents for chronic kidney diseases based on a novel action mechanism and it is useful for treating and preventing chronic kidney diseases with fewer side effects.

## Claims

1. A therapeutic agent for chronic kidney diseases, which comprises as an active ingredient a compound capable of inhibiting Na⁺/Ca²⁺ exchanger 1 (NCX1).

2. A therapeutic agent for chronic kidney diseases, which comprises as an active ingredient a compound capable of specifically inhibiting Na⁺/Ca²⁺ exchanger 1 (NCX1).

3. A therapeutic agent for chronic kidney diseases, which comprises as an active ingredient a 2-phenoxyaniline derivative of Formula (1): 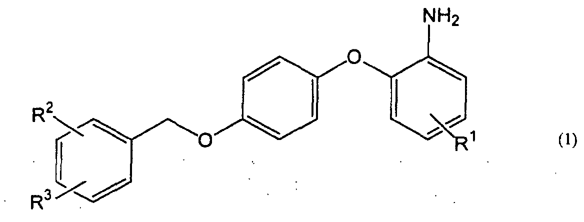 [wherein R¹ represents a hydrogen atom or a C₁-C₆ alkoxy group, R² represents a halogen atom or a nitro group, and R³ represents a hydrogen atom or a halogen atom] or a pharmaceutically acceptable salt thereof.

4. A method for treating or preventing chronic kidney diseases **characterized in** inhibiting Na⁺/Ca²⁺ exchanger 1 (NCX1).

5. Use of a compound capable of inhibiting Na⁺/Ca²⁺ exchanger 1 (NCX1) for protecting kidney in the treatment of chronic kidney diseases.
